Europäisches Patentamt

European Patent Office

Office européen des brevets

(11) Publication number: **0 589 487 A1**

## EUROPEAN PATENT APPLICATION

(21) Application number: 93116126.9

(22) Date of filing: **14.03.90**

(51) Int. Cl.5: **C12P 21/08**, C12N 5/16, G01N 33/53, G01N 33/84, C07K 15/28

This application was filed on 06 - 10 - 1993 as a divisional application to the application mentioned under INID code 60.

(30) Priority: **14.03.89 US 324392**

(43) Date of publication of application:
**30.03.94 Bulletin 94/13**

(60) Publication number of the earlier application in accordance with Art.76 EPC: **0 463 110**

(84) Designated Contracting States:
**AT BE CH DE DK ES FR GB IT LI LU NL SE**

(71) Applicant: **BOARD OF REGENTS OF THE UNIVERSITY OF NEBRASKA**
**3835 Holdrege Street**
**Varner Hall**
**Lincoln NB 68503(US)**
Applicant: **BIONEBRASKA, INC.**
**3920 Cornhusker Highway**
**Lincoln, NS 68504(US)**

(72) Inventor: **Wagner, Fred W.**

**Route 1,**
**Box 77B**
**Walton, Nebraska 68461(US)**
Inventor: **Wylie, Dwane E.**
**5641 Hickory Crest Road**
**Lincoln, Nebraska 68516-2452(US)**
Inventor: **Schuster, Sheldon M.**
**910 Southwest 112th Street**
**Gainesville, Florida 32607(US)**
Inventor: **Coolidge, Thomas R.**
**Beebe Hill Road**
**Falls Village, Connecticut 06031(US)**
Inventor: **Song, Pill-Soon**
**5930 Woodstock**
**Lincoln, Nebraska 68512(US)**
Inventor: **Parker, William**
**1020 East Street,**
**Apt. E**
**Lincoln, Nebraska 68508(US)**

(74) Representative: **Türk, Gille, Hrabal, Leifert**
**Brucknerstrasse 20**
**D-40593 Düsseldorf (DE)**

(54) Monoclonal antibodies for small organic molecules.

(57) The present invention is directed to monoclonal antibodies and fragments thereof for immunoreaction with organic compounds having a molecular size of no more than about 15 atoms in length and being a linear or branched aliphatic molecule or a small peptide. The invention is as well directed to hybridomas which produce the monoclonal antibodies, to immunogen compounds of the organic compound which cause development of the appropriate mammalian antibody response and to methods for producing antibody fragments. The invention is further directed to methods for detecting, removing, adding or neutralizing the organic compounds in biological and inanimate systems through the use of the monoclonal antibodies or fragments thereof.

## Cross-Reference to Related Applications

This is a continuation-in-part of U.S. Application Serial No. 07/324,392, filed March 14, 1989.

## Technical Field

This invention relates to: novel methods for detecting, removing, neutralizing or adding minute quantities of small moieties such as small organic molecules; monoclonal antibodies or fragments thereof that are immunoreactive with the small moieties; and hybridomas and other methods for production of the monoclonal antibodies or fragments thereof.

## Background of the Invention

Small chemical moieties can and often do affect the environment and biological systems. These effects become astounding when it is realized that minute quantities of small moieties are involved. Moreover, the presence or absence of low concentrations of small moieties in the environment can have long term consequences. Minute quantities of small organic molecules can regulate, influence, change or toxify the environment or biological systems.

The detection, removal, addition or neutralization of such minute quantities constitutes a focal point for continued research in many fields.

Small organic molecules such as cleaning fluids (e.g. trichloroethylene), as well as many pesticides and herbicides have small business, agricultural and industrial applications. The former are used in processing, formulating, cleaning and purifying while the latter retard infestation by vermin, insects and undesired plants. However, these molecules also find their way into ground water and subsequently contaminate water used for consumption, agricultural and industrial purposes. Hence, efficient and accurate identification of minute concentrations of small organic molecules in drinking water or other media would be an important step toward their control.

Cosmetic formulations, perfumes, and other proprietary products often contain minute levels of certain small organic compounds. The appropriate selection and mixture of these compounds is the secret of the perfumer's art. Hence, determination of the concentrations and identities of these compounds could serve as a means for cosmetic control or for cosmetic design.

Many foods contain minute quantities of small organic compounds. These compounds contribute to the flavor notes and odor of the foods. For example, ethyl butyrate and limonene contribute to the fresh flavor notes so characteristic of freshly squeezed orange juice. Hence, determination of the concentrations and identities of such compounds within foods and the isolation and purification of the same would help advance food design and screening.

Removal of minute quantities of small moieties from biological or inanimate systems carries many implications.

It would, therefore, be highly desirable to identify and control minute quantities of helpful or harmful small moieties in aqueous biological or inanimate systems. In most contexts, however, the detection, removal, addition or neutralization of small moieties is a difficult, time consuming, expensive and often unfeasible if not impossible task. Other compounds often mimic the small moieties and interference with measurements will result. Moreover, the detection methods employed today are usually not sufficiently sensitive at the minute quantities under consideration here. Consequently, it is desirable to develop reliable, quick and economic methods which can be used in the lab or the field for accurately identifying and controlling minute quantities of small moieties in aqueous systems.

Antibodies would seem to be uniquely suited for this task. Their high degree of specificity for a known antigen would avoid the interference caused by contaminants. Their sensitivity in the picomolar or lower range would accurately and efficiently target and detect the minute levels.

Monoclonal antibodies, of course, come to mind as especially suited agents for practice of this technique. Since Kohler and Milstein published their article on the use of somatic cell hybridization to produce monoclonal antibodies (Nature 256:495 (1974)), immunologists have developed many monoclonal antibodies which strongly and specifically immunoreact with antigens.

Notwithstanding this suggestion, the conventional understanding about immunology teaches that antibodies against small moieties cannot be developed. The mammal immunization step, which is key for the production of monoclonal antibodies, requires a molecule that is large enough to cause antigenic reaction. Medium sized molecules (haptens), which are not of themselves immunogenic, can induce immune reaction by binding to an immunogenic carrier. Nevertheless, immunologists view small molecules such as small

2

organic molecules as not large or structurally complex enough to elicit an antibody response. One theory appears to hold that electron rich rings such as those associated with benzene and carbohydrates are needed at a minimum to cause immunogenicity. V. Butler, S. Beiser, Adv. Immunol., 17, 255 (1973). The molecular size and complexity of an organic small moiety is thought to be insufficient for eliciting an antibody response. To date, therefore, no monoclonal antibodies which immunoreact with small moieties per se have been reported in the literature.

Several immunologists have reported production of monoclonal antibodies to metallic ion chelates. For example, in U.S. Patent No. 4,722,892, monoclonal antibodies are disclosed which immunoreact with a complex of a chelating agent, such as ethylene diamine tetracetate (EDTA), and a heavy metal such as indium. In EPO Patent Application 0235457, monoclonal antibodies that immunoreact with a chelate of gold cyanate and carbonate coating are disclosed. In these instances, however, the monoclonal antibodies bind with the metal chelate complex rather than the bare metallic ion itself. Disadvantages of these methods include: the complicated reagents involved in detection, lack of simple tests that discriminate among antigens, cross-reactivity with chelates of other antigens and cross-reactivity with the chelate itself.

Other instances of monoclonal antibody combinations with metals involve metal tags. The metal chelates are bound to the antibody at a site remote from the antigen binding site or sites. The metal or metal chelate is not the antigen. Instead, it is a tag to indicate the presence of the monoclonal antibody when it reacts with its specific antigen. See for example, V.P. Torchilian et al., Hybridoma, 6, 229 (1987); and C.F. Meares, Nuclear Medical Biology, 13, 311-318 (1986).

It is therefore, an object of the invention to develop monoclonal antibodies and fragments thereof that immunoreact with small moieties per se. It is another object of the invention to develop methods for detecting or neutralizing small moieties within, adding small moieties to, or removing small moieties from biological or inanimate systems through the use of the monoclonal antibodies and fragments thereof. Further objects include development of hybridomas which produce the monoclonal antibodies, development of immunogen compounds for generation of antibody reactivity to the small moieties and development of methods for production of antibody fragments. Yet another object is the development of monoclonal antibodies and fragments thereof that are capable of discriminating very similar small moieties.

## Summary of the Invention

These and other objects are achieved by the present invention which is directed to monoclonal antibodies and fragments thereof for immunoreaction with small moieties. The invention is as well directed to hybridomas which produce the monoclonal antibodies, to immunogen compounds of the small moieties which cause development of the appropriate mammalian antibody response and to methods for producing antibody fragments. The invention is further directed to methods for detecting, removing, adding, or neutralizing the small moieties in biological and inanimate systems through the use of the monoclonal antibodies or fragments thereof.

The advantages of the invention include among others: the lack of complication by additional reagents, a preference for a high binding constant with a group of small moieties having similar coordination chemistry and electron configuration and a lack of cross-reactivity with other classes of antigenic materials and other classes of small moieties, an especial preference for a high binding constant with a particular small moiety and a lack of cross-reactivity with all other small moieties, and lack of cross-reactivity with test reagents.

The monoclonal antibody and fragments thereof immunoreact with a small moiety per se. The state of the small moiety during this immunoreaction is one of non-coordination with any other substance; in other words, in a preferred state it is bare or exposed. The monoclonal antibody or fragment thereof will also undergo immunoreaction with small moiety when it is in a partially exposed state as well. Preferably, the monoclonal antibody and fragments thereof exhibit high specificity toward a group of small moieties with similar coordination chemistry and electron configuration and little cross-reactivity for other classes of small moieties. Especially preferably, the monoclonal antibody and fragments thereof exhibit a substantially high degree of specific immunoreactivity toward a particular small moiety and little cross-reactivity with all other small moieties.

The monoclonal antibody or fragment shows a discrimination between a particular group or individual small moiety and other groups or other individual small moieties that constitutes at least about a $10^2$ difference, preferably a $10^3$ to $10^5$ difference, in the respective dissociation constants. Preferably, the monoclonal antibody is a member of the immunoglobulin G, A or E classes and has an association constant for the small moiety that is about 10,000 fold greater than the association constant for the immunogen compound without the small moiety. When the monoclonal antibody displays immunospecificity for a particular member of a group of very similar small moieties, its relative association constant for such a particular small moiety will be about 10,000 fold greater than that for the other small moieties of such a group.

The invention includes as well fragments of the monoclonal antibody such as the $F_{ab}$, $F_{(ab)}$' fragments, and the individual heavy and light chains of the monoclonal antibody. Fragments and functional segments of the individual chains, especially the entire variable region fragment and segments thereof, and mimetic variable region segments of the monoclonal antibody (i.e., polypeptides having sequences which incorporate segments of the monoclonal antibody variable region or constitute variations of that variable region) are also contemplated.

The hybridoma of the invention, which produces the monoclonal antibody, is formed from immune cells that are sensitized toward the small moiety or class of similar small moieties. The formation is accomplished by fusion of an immortal mammal cell line and mammal immune cells from a second mammal previously immunized with the immunogen compound which contains the small moiety. Selection of the appropriate hybridoma is determined by cross-screening the secreted monoclonal antibody against the small moiety and against controls which incorporate the small moiety or very similar congeners.

Fragments of the monoclonal antibodies including $F_{ab}$, $F_{(ab)}$', the heavy and light chains and fragments or segments thereof and mimetic variable region segments as well as the monoclonal antibodies themselves can be produced by recombinant cDNA techniques for generating plasmid libraries containing a repertoire of DNA sequences taken from the antibodies as described in the following sections. Selection of the desired fragment or monoclonal antibody is accomplished by testing the expression products of the library of recombinant cells containing the recombinant expression vectors. The test involves assaying for immune reaction between the expression product and an immunogen compound or a combination of antigen probe and the small moiety.

The immunogen compound of the invention is composed of a biopolymer carrier, a spacer arm covalently bonded to the carrier and the small moiety which is coordinated or covalently bonded to the spacer arm. The spacer arm is semi-rigid and has at least one small moiety coordination site. This arrangement maintains the small moiety in at least a partially exposed state and prevents substantially complete inclusion or chelation of the small moiety by spacer arm and/or carrier. Preferably, the spacer arm coordination with the small moiety is monodentate.

The biopolymer carrier may be a polysaccharide, a synthetic polyamide or preferably a protein. Preferred classes include blood or tissue proteins.

The spacer arm is no more than about 25 atoms in length. It is composed of one of three classes: an oligopeptide, an aliphatic compound or an aliphatic fragment. The first two are each substituted with no more than about 2 pendent Lewis acid or base groups, and a coupling group for forming a covalent bond with the protein carrier. The latter is substituted by a coupling group for forming a covalent bond with the protein carrier, and a carboxylic acid, aldehyde, hydroxyl, mercapto, amine or other group adapted for carbon-carbon bonding with, or covalently bonded to, the small moiety. For each class of spacer arm, the coupling group is an amine, carboxylic acid, aldehyde, hydroxyl or mercapto group. The latter class of spacer arm is appropriate when the small moiety is an organic compound.

The small moiety has a molecular size of no more than about 15 to 20 angstroms in length. Included are linear or branched aliphatic organic molecules having a molecular size of no more than about 15 atoms in length. Organic small moieties having aromatic rings, especially non-electronegatively substituted aromatic rings, can as well be detected, neutralized, added or removed according to the invention.

Organic compounds of special mention as the small moiety include aliphatic compounds, linear organic compounds, small peptides, saccharides, fats, linear organic compounds which may be substituted by polar groups, pesticides, herbicides, toxic halogenated organic compounds and aromatic compounds. The organic compounds, especially the aliphatic compounds, may be optionally substituted by halogens and other groups such as esters, ethers, hydroxyls, amides, sulfides, sulfones, sulfates, sulfhydryls, nitros, nitriles and the like. The aromatic compounds may be substituted by groups such as esters, ethers, hyroxyls, amides and sulfides.

The methods according to the invention utilize the monoclonal antibody or a fragment thereof for detection, removal, neutralization or addition of the small moiety respectively in, from, within or to a liquid or gaseous medium. These methods utilize features such as monoclonal antibody or fragment immobilization, small moiety immobilization, competitive binding, means employing an oscillating probe, means for micromagnetic binding, anti-sera antibody for the monoclonal antibody or fragment, enzyme tags, radioactive tags, spectrometric tags and other physiochemical methods used to monitor antigen(i.e., small moiety)-antibody interactions.

Methods for detection that are based upon immobilization indicate the presence of the small moiety-monoclonal antibody (or fragment thereof) complex by known immunologic assay techniques. In a step either before or after formation of the complex, the small moiety (or complex) may be coordinated with an immobilized antigen probe for the small moiety. The antigen probe may be a spacer arm or a peptide or protein having a complexing group including a sulfhydryl, carboxyl, carbonyl, amino, hydroxyl or amide group or any combination thereof. When the probe formula is the same as the spacer arm formula, the probe may be, but need not be, the same structure as the spacer arm of the immunogen compound used to develop the monoclonal antibody. The probe will hold the small moiety in at least a partially exposed state. Non-immobilized materials are then removed from the mixture holding the immobilized probe-small moiety or immobilized probe-complex, and if the antibody or fragment has not been added, it is added at this point to form the immobilized probe-complex. Immunoassay completes the steps for this detection method.

Methods for detection that are based upon an immobilized monoclonal antibody or a fragment thereof utilize tagged substances that bind with monoclonal antibody, fragment or complex. A radioactive version of the small moiety or a similar tagged form thereof can also be used. The tags include fluorescent, colorimetric, enzymatic, tagged general anti-sera antibodies for the monoclonal antibody or fragment, and other spectrally active groups that can be coordinated or bonded to the monoclonal antibody, fragment, complex or small moiety. Preferred tags for the antibody include color producing enzymes and anti-animal species antibodies. A preferred tag for the small moiety is a an antigen probe containing a spectrally active group. In a first step, the immobilized monoclonal antibody or a fragment thereof is combined with the medium suspected of containing the small moiety. After removal of the non-immobilized components, an aliquot a tagged substance is added. It binds to immobilized complex and measurement of the tagged complex will determine the concentration of unknown small moiety.

Methods for detection that are based upon an oscillating probe utilize either an immobilized probe for the small moiety or preferably immobilized monoclonal antibodies or fragments thereof. This method measures the change in frequency of an oscillating surface as a function of the change in weight of that surface due to the binding of the non-immobilized small moiety or monoclonal antibody (or fragment thereof). In the preferred method the monoclonal antibodies or fragments thereof are immobilized on the surface of a high frequency oscillating probe. The probe is placed into a medium containing an unknown quantity of small moiety. Binding of the small moiety to the immobilized monoclonal antibody or fragment thereof will change the oscillation frequency of the probe. Hence, the degree of change will indicate the level of small moiety present.

In an especially preferred version of this method, a fixed support is utilized. Here, either the oligopeptide or the monoclonal antibody (or fragment) is immobilized on the fixed support. The method is then conducted as related above.

The invention, in addition, contemplates methods for small moiety removal from, small moiety neutralization within or small moiety addition to biological or inanimate systems. For all methods, an effective amount of the monoclonal antibody or fragment thereof is combined in some fashion with at least part of the system. Pursuant to the removal method, immunoconjugated monoclonal antibody(or fragment)-small moiety is removed by separation means such as immunoprecipitation, immobilization, chromatography, filtration, magnetic attraction, photolytic binding, affinity binding and the like. Pursuant to the neutralization method, the immunoconjugated monoclonal antibody(or fragment)-small moiety remains in the system until it is removed by non-specific means. Pursuant to the addition method, the immunoconjugated antibody(or fragment)-small moiety also remains in the system and the small moiety is actively incorporated or otherwise used therein.

When the system treated in the foregoing methods is inanimate such as contaminated water or industrial waste, the monoclonal antibody or fragment may be immobilized on a solid support or may be capable of binding, reacting or complexing with another substance that is immobilized or immobilizable. These steps encompass affinity binding of an enzyme or antisera antibody (for the monoclonal antibody) with the monoclonal antibody or with a tag bound to the monoclonal antibody, magnetic separation through a tag containing iron, photolytic binding agents bound to the antibody such that photolysis will cause separation, and removal by binding or partition on a solid support especially as applied to any of the foregoing substances. Use of the probe in these instances is optional. This method is preferred as a means for removing heavy metals from aqueous environments. Removal of undesirable metals such as lead, mercury and cadmium as well as desirable metals such as gold, silver, platinum and palladium is contemplated.

When the system treated in the foregoing methods is biological, the monoclonal antibody or fragment thereof may be combined with a pharmaceutically acceptable carrier. Preferably, the monoclonal antibody or fragment thereof will not of itself cause an undesirable immune response of the biological system. The biological systems contemplated according to the invention include unicellular organisms, multicellular simple organisms, cellular component systems, tissue cultures, plants and animals, including mammals.

The present invention also contemplates methods for removing radioactive compounds from human fluids such as blood, serum or lymph by utilization of immobilized monoclonal antibodies or fragments thereof. An extracorporeal shunt placed in the patient permits removal of the body fluid and its reintroduction. Passing the body fluid extracorporeally through a bed of immobilized monoclonal antibody or fragment thereof accomplishes the desired removal. An especially preferred method is use of an extracorpeal shunt for blood in combination with a monoclonal antibody or fragment tagged with a substance that is immobilizable. Such substances include substances for enzymes, e.g., sulfanilamide and carbonic anhydrase, ligands and proteins, e.g., avidin and biotin, and magnetic compounds, e.g., organic iron derivatives that will be attracted to an electromagnetic source. The monoclonal antibody (or fragment) tagged with immobilizable substance is injected into the patient through the shunt. After circulation and complexation with the small moiety in vivo, the shunt is vented and the complex carrying the immobilizable substance is removed extracorporeally from the shunted blood by the appropriate immobilization technique such as affinity binding or magnetic separation.

When a method for adding a monoclonal antibody (or fragment)-small moiety conjugate to a biological or inanimate system is contemplated, the monoclonal antibody or fragment will preferably be bifunctional. The second binding site of the monoclonal antibody or fragment will be reactive with a selected component of the system. That component may be a complex organic molecule, living cells, selected tissue of a tissue culture or a selected tissue of an animal. In this method, the small moiety will exert a desirable action upon the component of the biological or inanimate system targeted.

The present invention also contemplates a kit for assaying the presence and quantity of small moiety in a biological or inanimate system. The kit includes aliquots of monoclonal antibodies or fragments thereof in the appropriate buffer, as well as a fixed support with antigen probe for absorption of the small moiety, washing solutions, reagents such as enzyme substrates, and antisera conjugated to a detectable substrate which antisera are generally reactive with all antibodies produced by the source from which the monoclonal antibodies or fragments thereof are derived.

This invention is directed to the use of the genes for the antibodies or for coding for any portion of the antibody molecule or derivatives thereof. Synthetic genes created for the purpose of coding for the antibodies or any portion thereof are included. The invention also includes the expression of these genes in any bacteria, yeast, plant, animal, virus, fungi, or any other organisms for any purpose. Such purposes include treatment of toxic waste soil, water or effluent; mining of metals using these organisms; bioremediation; medical uses; and agricultural uses.

The invention is further directed to a cocktail of the monoclonal antibodies or fragments thereof which immunoreacts with a group of different small moieties. This cocktail can be used to remove, for example, small organic molecule contaminants from a medium.

The invention is also directed to a purified class of polyclonal antibodies having high specificity for a group of small moieties with similar coordination chemistry and electron configuration. These polyclonals constitute the sensitized antibodies present in the body fluids of a mammal immunized with the immunogen compound according to the invention. Collection of the immunized animal's fluid, e.g., blood, followed by affinity column purification using the immunogen compound as a probe will concentrate all the antibodies reactive toward the small moiety. Although this animal's immune cells can be used to form the hybridomas discussed above, at this stage of the process, the animal will produce the polyclonals of the invention. Typical animals useful in this regard are goats, sheep, pigs and cows.

## Detailed Description of the Invention

The monoclonal antibodies and related fragments of the present invention are key to the development of methods for detecting, adding, neutralizing or removing minute quantities of small moieties. Until the present invention, it was not possible to produce antibodies to to small linear organic compounds, especially those without rings. The novel techniques for incorporating small moieties into immunogen compounds and for administering these immunogen compounds to immune cell hosts allow production of the desired, immunospecific monoclonal antibodies according to the invention. These methods are believed to constitute an advancement in the understanding of immunology.

Although not intended as a limitation of the invention, it is now believed that mammalian immunogenic reactivity can be elicited by small moieties. While they are smaller than the recognized epitopal size of approximately 20-25 angstroms, the small moieties nevertheless can epitopally bind.

Notwithstanding these beliefs, the invention contemplates monoclonal antibodies (and fragments thereof) to small moieties, the hybridomas therefor, the immunogen compounds for carrying the small moieties and inducing immunogenicity, and methods for detection, addition, neutralization or removal of small moieties using the monoclonal antibodies or fragments thereof.

Monoclonal Antibodies

The monoclonal antibodies of the invention are mammalian immunoglobulin proteins, preferably IgG, IgA, or IgE classes, which have strong affinity constants for specific small moieties and/or classes thereof. They are characterized by selective immunoreactivity with a class of small moieties having similar coordination chemistry and electron configuration. Preferably, they exhibit high specificity toward a particular, individual small moiety and a substantially lower immunoreactivity with other small moieties including those with similar electron configurations. The monoclonal antibodies have an association constant for the selected small moiety that is at least about 100 fold and preferably at least about 10,000 fold greater than the association constant for the other small moieties.

With respect to small organic molecules, the IgG, IgA and IgE classes of monoclonal antibodies of the present invention exhibit discriminatory dissociation constants of at least about $10^{-6}$. Examples include monoclonal antibodies that are selective for glutathione, serine ester, N-blocking groups for amino acids, trichloroethylene, polychlorinated biphenyl, ethyl mercaptan, chlordane and hydrophobic residues.

The monoclonal antibody fragments, single chains, fragments of the chains and mimetic variable regions function like the monoclonal antibodies of the invention. They display an ability to complex with the small moieties as described above. In the remaining discussion, it will be understood that mention of the monoclonal antibodies includes antibody fragments, single chain fragments as well as the mimetic variable regions.

Immunogen Compounds

The immunogen compounds for generation of the specific immunogenicity of the monoclonal antibodies are based upon the hapten-carrier concept. The present invention, however, broadens this concept so that the hapten is coordinated at the end of a spacer arm covalently bonded to the carrier. The spacer arm is adapted so as to be semi-rigid and to hold the small moiety in an exposed position relative to the carrier. This arrangement is also adapted to maintain the small moiety in a substantially exposed and preferably, essentially completely exposed state. These factors combine substantially to avoid chelating, encovering or inclusion of the small moiety by the spacer arm and/or the carrier.

The spacer arm, as characterized above, may be an oligopeptide, an aliphatic compound, or an aliphatic fragment. In the latter two instances, the aliphatic compound or fragment may be covalently bonded to the carrier by means of a Schiff base reaction with an aldehyde group, an amide reaction with an amine or carboxylic acid group using a peptide activator such as carbodiimide, acid chloride and the like, an ester reaction with a hydroxyl or carboxylic acid group using a Schotten Bauman reaction, or azide or acid catalysis reaction, a sulfide reaction using a sulfide coupling agent, or other known coupling reactions for joining organic molecules to proteins. See for example Kabat, E.A., Structural Concepts In Immunology and Immunochemistry, 2nd Ed., Holt, Rinehart and Winston, New York, 1976 (a review text of such methods) and Jaime Eyzaguirre, Chemical Modification of Enzymes: Active Site Studies, John Wiley & Sons (1982), the disclosures of which are incorporated herein by reference. The oligopeptide, aliphatic compound or fragment will contain backbone groups which provide semi-rigidity to the spacer arm. Preferred groups for developing this semi-rigidity include peptide bonds, olefin bonds, olefinic conjugated systems, ester groups and enone groups. Optionally, and especially where immunogenicity of the small moiety appears difficult to generate, one or more aromatic rings can be incorporated into the spacer arm to stimulate the development of an immune response.

In general, the oligopeptide spacer arm has the following formula:

- X - (R) - Y

wherein X is a coupling group that will bond to the carrier, R is one or more amino acid residues and Y is the Lewis Acid or Base group(s) for small moiety coordination.

In general, the aliphatic compound or fragment spacer arm has the following formula:

- X - (Q) - Z

wherein X is a coupling group that will bond to the carrier, Q is a semirigid aliphatic moiety containing ester, amide, keto, olefin or aromatic groups and the like, and Z is a Lewis acid or Base group(s) for small moiety coordination or is a group that will form a covalent carbon bond with an organic small moiety to form a mimic.

When the small moiety is a small organic molecule, the spacer arm will be covalently bonded or bondable to it. The resulting compound will be a spacer arm-small moiety mimic. The semi-rigidity of the spacer arm portion of the mimic holds the small moiety portion in a position exposed and remote from the carrier. Preferred classes of spacer arm mimics for this aspect of the invention include aliphatic carboxylic acids, amines and aldehydes having semi-rigid backbones and having tails which duplicate the structure of the small moiety. For example, when the small moiety is trichloroethylene, an appropriate spacer arm - mimic is N -(trichloroacryoyl) glycine. In general, the structure of the spacer arm-mimic is as follows:

$$- X - (Q) - A - M_s$$

wherein X and Q are defined as given above, A is a bond or group attaching $M_s$ to Q and $M_s$ represents the organic molecule which is acting as the small moiety. An example of this technique as applied to organic molecules is use of glutathione. As discussed in detail below, monoclonal antibodies specific to glutathione have been made.

The spacer arm mimic can as well act as a tagged small moiety. It participates in the methods for detection that employ immobilized monoclonal antibody. Spectrally active groups can be joined to the spacer arm mimic or incorporated therein to provide a discernable, detectable signal. Such groups include fluorescent groups, colorimetric groups, U.V. groups and others known in the art.

The carrier of the immunogen compound is a large biopolymer that is known to participate in the development of hapten antigenicity. Blood serum proteins, amylopectins, polysaccharides, fetal serum components, biologically acceptable natural and synthetic proteins and polyamides such as polyglycine can serve as the carriers. Preferred carriers include serum and tissue proteins. Examples are keyhole limpet hemocyanin (KLH) and bovine serum albumin (BSA). Other examples include ovalbumin and chicken gamma globulin. These carriers have sites for coordinate bonding of the spacer arm. Such sites are preferably populated by amine groups, carboxylic acid groups, aldehyde groups and/or alcohol groups.

Production of Hybridomas

The production of hybridomas according to the invention generally follows the Kohler, Milstein technique. Many of the small moieties, however, toxify the mammalian system being used as a source of immune cells. This effect makes it important to determine the highest allowable dose of small moiety and/or immunogen compound that can be used over a substantially long period of time without killing the host.

Pursuant to the Kohler, Milstein technique, immunization of the mammalian host is accomplished within this dose parameter by subcutaneous or intraperitoneal injection of the immunogen compound in adjuvant. Administration is repeated periodically and preferably for at least four injections. Three days before the spleen is removed, a priming injection of immunogen compound is again administered.

At this stage of the procedure, the immunized mammal also secretes into its blood stream polyclonal antibody to the small moiety. Isolation and concentration of the polyclonal by known affinity binding techniques produces the polyclonal composition of the invention. Goats, pigs, rabbits, cows, horses, sheep and the like are useful as mammalian sources for the polyclonal and hybridoma productions.

After their separation, the spleen cells are fused with immortal mammal cells such as mouse myeloma cells using the techniques outlined by Kohler and Milstein. Polyethylene glycol (PEG) or electrical stimulation will initiate the fusions.

The fused cells are then cultured in cell wells according to culture techniques known in the art. Cellular secretions in the culture medium are tested after an appropriate time for the presence of the desired cellular products.

Selection Technique

The selection technique for identifying the appropriate monoclonal antibody is an important aspect for determining the immunospecificity desired according to the invention. The selection techniques according to the invention call for determining the binding affinity of the hybridoma cellular products against the small moiety and against cross-reactive controls. In particular, hybridoma culture fluid is tested in screening assays against the small moiety, the carrier, the carrier-spacer arm product and the immunogen compound as well as optionally against the spacer arm-small moiety coordinate. In instances where the small moiety is an organic compound, cross-reactivity controls against a spacer arm-mimic wherein the mimic tail includes the backbone of the small moiety but not the carbon substituents of the small moiety, may be included. This will eliminate cross-reactivity with the carbon backbone of the spacer arm-mimic.

Screening assays can be performed by immunoenzymatic-assay, immunofluorescence, fluorescence-activated cell sorter, radioimmunoassay, immunoprecipitative assay or inhibition of biological activity.

The hybridoma cultures selected will exhibit strong binding characteristics to the small moiety (and immunogen compound) and exclude binding with the spacer arm-carrier product and with the carrier itself.

Following the identification of cell cultures producing the desired monoclonal antibodies, subcloning to refine the selected culture can be performed. These techniques are known to those skilled in the art. See for example James Goding, Monoclonal Antibodies: Principles and Practice, 2nd Edition, Academic Press, San Diego, CA 1986, the disclosure of which is incorporated herein by reference.

Briefly, the appropriately selected cell culture is separated into one cell units which are then recultured. The subclone cultures are then again tested for specific immunoreactivity, lack of cross-reactivity and the amount of monoclonal antibody secreted. Those subcultures exhibiting the highest amounts of secreted monoclonal antibody are chosen for subsequent pilot development.

Following the foregoing techniques, hybridomas producing monoclonal antibodies to mercury cation and to the group of lead, gold and mercury cations have been developed. These perpetual cell lines, designated 4A10 and 3H7 respectively are maintained in culture medium and in frozen medium at liquid nitrogen temperature at the laboratories of BioNebraska.

The immunogenic host for these hybridomas was the BALB/c mouse and the fusion partner was the mouse myeloma cell line P3X63-Ag8.653. Immunizations were accomplished with the immunogen compound formed from KLH, glutathione and the metallic cation functioning as the small moiety in complete Freund's adjuvant.

Deposits of these hybridomas with the American Type Culture Collection (ATCC) depository were made on March 13, 1990 by the applicants. The respective ATCC deposit numbers for the foregoing BioNebraska cell lines respectively are 4A10B4, ATCC number HB10381 and 3H7G5, ATCC number HB10382. Applicants accept and will designate the provisions of the Budapest Treaty for these deposits. Applicants agree that the hybridomas will be available upon publication or issuance of this patent application. Applicants agree that upon request the Commissioner of Patents shall be entitled to direct submission of the hybridomas to a depository or other recipient he shall designate. Applicants certify that the hybridomas are viable. Applicants certify that the hybridomas are also available from BioNebraska, Inc. pursuant to and under the provisions of the Budapest Treaty for microorganism deposits. Applicants agree that the foregoing conditions apply also to their own maintenance of the hybridoma cultures. Applicants assure that they will maintain their hybridoma cultures for at least thirty years and will request the same of the international depository.

Fragments and Recombinant Method

Fragments of the monoclonal antibodies, such as the $F_{ab}$ and $F_{(ab)}'$ fragments can be generated by well-known techniques. For example, cleavage of the monoclonal antibody with papain enzyme will produce the $F_{(ab)}'$ fragment while cleavage with papaverine will produce the $F_{ab}$ fragment. See "Antibodies. A Laboratory Manual," E. Harlow and D. Lane, Cold Spring Harbor Laboratory, 1988.

A recombinant method for generating the fragments of monoclonal antibodies as well as the monoclonal antibodies themselves utilizes the techniques for generating plasmid libraries containing antibody repertoires such as the techniques developed by Lerner et al., Science, 246, p.1275 (1989) and Winter et al., Nature, 341, 544 (1989). According to these methods, a library of sensitized immune cells is produced by immunizing the mammal with immunogen compound. Restriction and isolation of the set of antibody genes from the library of immune cells of the mammal forms the set of genes for creation of the gene library. This set of genes can be used without further manipulation or can be further restricted to yield gene fragments for the $F_{ab}$, $F_{(ab)}'$, single chain, single chain fragments and mimetic variable regions. The library of antibody genes is spliced into an appropriate vector and the recombinant vector inserted into a compatible host such as E. coli. The recombinant host cells produce a library of monoclonal antibodies or fragments thereof pursuant to the expression code provided by the recombinant vector. By application of the Winter and Lerner techniques to the recombinant method of the present invention the library of gene fragments for the $F_{ab}$, $F_{(ab)}'$, single chain, single chain fragment, or mimetic variable region segments are produced in vivo. These methods permit production of the appropriate monoclonal antibody library as well.

Following preparation of the repertoire of recombinant cells, the cellular library is cloned and the individual clones screened for the monoclonal antibody or fragment thereof that selectively reacts with small moiety. Pursuant to the Lerner and Winter techniques, any of the known methods for screening recombinant DNA libraries such as radioassay of colony transfers. The initial screen employs the immunogen compound or a combination of the antigen probe and the small moiety as a screening reagent. Cross-reactivity with the carrier and spacer arm is eliminated by selection of those monoclonal antibodies or fragments from the first pass that do not react in a second and third assay with the carrier arm or carrier spacer arm moieties alone.

Through these techniques, the need to perform cellular fusions and grow hybridomas is eliminated. The techniques streamline the small moiety monoclonal antibody production and avoid tedious hybridoma formation.

Methods of Application

According to the invention, the monoclonal antibodies can be used to advantage for detection, neutralization, addition or removal of small moieties from biological or inanimate systems. These methods apply to qualitative and quantitative analyses or detection of such embodiments as minute concentrations of herbicides, pesticides and toxic small organic molecules in aqueous liquid systems and minute quantities of organic molecules in there biological or environmental systems or in such compositions as perfumes, cosmetics, pharmaceuticals, health care products, skin treatment products, pesticides, herbicides, toxic solvents used in the production of semi-conductor and integrated circuit components and production materials for electronic components. In each application, the presence of minute quantities of small organic materials could constitute deleterious contaminants. Their ready and early detection will avoid later production or regulatory set-backs.

Alternatively, the presence of minute quantities of small moieties in certain instances may be desirable. For example, the presence of small organic molecules in food products, cosmetics and perfume and the presence of inorganic moieties in such mixtures as doping materials for semi-conductors and integrated circuits contributes to the properties of the product. Quality control of the presence and concentration of these small moieties is essential for the functioning of the product. The detection methods of the invention enable ready and early measurement of the presence of such moieties and avoid later production or regulatory difficulties.

In the broadest aspect, the method for detection utilizes the complexation reaction of the monoclonal antibody or fragment thereof and the small moiety or group thereof and an assay technique for the presence of the complex. The complex may be immobilized before or after its formation or may be treated in similar fashion to isolate it from the uncomplexed reagents and antibody. Assays such as ELISA, radioimmunoassay, sandwich, antisera antibody reaction, enzyme coloration reaction and tagging can be use for quantification.

The small moieties in biological or inanimate systems can also be removed by methods according to the invention, especially those small moieties constituting the embodiments mentioned above. In the main, mixture of the monoclonal antibodies or fragment thereof with the biological or inanimate system followed by treatment with an immobilized or immobilizable substance that is capable of binding or reacting with the antibodies, fragments and/or complexes will remove the small moieties.

The immobilization of the monoclonal antibodies or fragments thereof can also effect removal. Such immobilization can be accomplished by techniques known to those of skill in the art. See, for example, Affinity Chromatography, C.R. Howe & P.D.G. Dean, John Wiley & Sons, London 1974, the disclosure of which is incorporated herein by reference. Removal is accomplished by passing the system suspected as having the small moieties over the immobilized monoclonal antibodies designed to be specific for the small moiety or group of small moieties sought to be removed.

A further application of this removal method involves the use of a magnetic, affinity or photolytic agent bound to or bindable a non-active portion of the monoclonal antibody or fragment thereof. The agent permits removal of the complex of antibody (or fragment) through binding, coordination reaction or other physiochemical process designed to separate and/or immobilize the complex. Examples include affinity binding, e.g., with avidin and biotin or carbonic anhydrase and sulfanilamide or photolytic binding, e.g., with azide photoreaction with an amine group containing column, and with an iron containing agent that will separate magnetic attraction to a magnetic source.

An advantage of this method is the removal of undesirable small moieties in the presence of similarly structured desirable chemical compounds. For example, whole blood from a patient can be removed from the patient, optionally filtered to return the cellular blood components to the patient, and the serum or blood passed over immobilized monoclonal antibodies. Alternatively, the soluble monoclonal antibody or fragment can be tagged with a magnetic agent that will allow separation of the complex from extracorpeal blood by magnetic attraction means after the antibody has been circulated through the patent. The purified serum or blood can then be returned to the patient.

Additionally, the recombinant methods discussed above provide a means for sustained reproduction of the monoclonal antibody or fragment thereof within systems where the small moiety is to be removed or otherwise treated. The recombinant cells, for example, algae, E. coli, fungi, or iron bacteria, are cloned with the appropriate genetic machinery for expression of the monoclonal antibody or fragment thereof, as for example, an intracellular constituent within the cytoplasm. The cells have an ability to absorb the small moiety, so that the small moiety becomes bound to the monoclonal antibody or fragment thereof within the cytoplasm. Inoculation of the recombinant cells of the system into containing the small moiety, culturation of the recombinant cells within the system and harvest of the nature culture produces removal and/or treatment of the small moiety.

Methods for adding small moieties to biological or inanimate systems focus on the delivery of the small moiety to a particular site. In this instance, the monoclonal antibodies will be bifunctional. The second binding site will be adapted to complex with a selected site within the biological or inanimate system. In this fashion, the monoclonal antibody-small moiety conjugate will deliver the small moiety to a specific site.

This method is particularly suited for heterogenous delivery processes. These processes enable the non-uniform concentration of the small moiety in a system that would otherwise cause its uniform or homogenous distribution. Examples include the delivery of anti-cancer compounds to target organs and tissues within a mammalian system, the delivery of radioactive compounds to specific organs and/or tissues in biological or inanimate systems and the delivery of small organic molecules to specific sites within a system. Fluid or semi-fluid flow of system ingredients would be preferred so that transport of the monoclonal antibody-small moiety conjugate can be rapidly made. The presence of a fluid medium, however, is not an important characteristic. Gels, semi-solidified systems and the like can be employed as long as some semi-fluid connection is present for diffusion of antigen and antibody.

When and immobilization and/or coordination of the small moiety is a step called for by the methods of detection, removal, addition or removal, the step can be accomplished through an antigen probe. The antigen probe generally binds or reacts with the small moiety and has the formula indicated above. Typically it is bifunctional so that after binding or reacting it will also immobilize, separate or otherwise distinguish the small moiety.

For administration of the monoclonal antibodies to biological systems, the antigenicity of the monoclonal antibodies themselves will preferably be minimized. Use of species-specific cell sources for generation of the hybridomas is an appropriate technique for minimizing the antigenicity of the monoclonal antibodies. Cross-reaction studies of the host and the monoclonal antibody can also be made to determine lack or minimization of monoclonal antibody sensitivity. A preferred means for avoiding adverse immune reaction is the use of the Fab or F(ab)$_2$ fragments of the monoclonal antibodies of this invention. These fragments do not contain the heavy chain tail primarily responsible for such immune reactions and are made by known methods.

In instances involving in vivo application, the dosage level and routes of monoclonal antibody administration will follow the judgment of the medical practitioner who is in an appropriate position to understand the needs and problems of the patient or mammal. In these situations, the dosage levels of monoclonal antibody compositions being administered will be consonant with the toxicity and sensitivity levels determined for the patient or mammal. The monoclonal antibody compositions will generally be combined for administration with a pharmaceutically acceptable medium such as water, alcohol, buffered aqueous medium, excipients, diluents and the like. Active transport agents can also be included. In general, the processes of administration for removal or addition of small moieties will maintain concentrations as high as possible so that the period for patient intervention is minimized. In each instance, consideration of the physiological characteristics of the small moiety will be important for determining the dosage levels and route of administration.

The invention will be further characterized by the following examples. These examples are not meant to limit the scope of the invention which has been fully set forth in the foregoing description. Variation within the concepts of the invention are apparent to those skilled in the art.

**Example 1**

**Mercury Cation Monoclonal Antibodies**

A. General Procedures

1. Generation of Hybridomas

Hybridoma antibodies have been produced with the spleen cells of BALB/c mouse that had received multiple injections of mercuric ions reacted with glutathione to produce a mercuric ion coordinate covalent compound, which was covalently bound to keyhole limpet hemocyanin ("KLH"). The KLH in complete Freund's adjuvant was utilized to assist in the elicitation of an immune response in the host animal. Glutathione is a three amino acid residue peptide having one reactive sulfhydryl group which forms a coordinate bond with mercuric ions.

Of 134 hybridomas isolated, four were determined to be producing monoclonal antibody specific for glutathione as set forth below in Table I. In addition, three other hybridomas (IFIO, 4AIO, and 3E8) were producing monoclonal antibodies that were strongly positive against glutathione-mercuric ions but negative against glutathione without mercuric ions (Table 1.). These three antibodies were subcloned by limiting dilution for further characterization. A fourth antibody (3F5), not included in Table 1, which appeared to be specific for glutathione but bound more tightly in the presence of mercuric ions, was also subcloned.

TABLE I

| ELISA Results From Initial Screening of Hybridoma Antibodies Reactive With Glutathione or Glutathione-Mercuric ions | | |
|---|---|---|
| Hybridoma | Glutathione | Glutathione-mercuric ions |
| IHII | I.2O2 | I.246 |
| 2A9 | I.O52 | O.758 |
| 3AI2 | 2.I27 | I.792 |
| 3H9 | 2.I34 | I.6O6 |
| IFIO | O.4O6 | I.I75 |
| 3E8 | O.4IO | I.O76 |
| 4AIO | O.4OO | I.IO4 |
| Negative[b] | O.456 | O.428 |

[a]Values are the absorbance at 405 nm shown by the specified hybridoma antibody in the ELISA.
[b]The value shown is the average absorbance at 405 nm of six wells on and ELISA plate that received culture fluid containing a monoclonal hybridoma antibody specific for dinitrophenol instead of culture fluid containing a mercuric ion specific monoclonal antibody in the first step of assay.

Only one positive subclone was obtained from hybridoma 3E8, and it subsequently lost its antibody-secreting ability. Several subclones secreting antibodies that were specific for mercuric ion were isolated from the other mercuric ion-specific hybridomas. The results of the analysis of these subclones and those from 3F5 with BSA-glutathione--mercuric ion and BSA-glutathione are shown in Table 2. All of the frozen hybridoma samples have been thawed from liquid nitrogen and assayed for persistence of antibody secretion after thawing.

TABLE 2

| ELISA Results from Hybridoma Subclones Specific for Glutathione or Glutathione-Mercuric ions | | |
|---|---|---|
| Hybridoma | Glutathione | Glutathione-mercuric ion |
| IFIO.A6 | O.289 | I.O48 |
| IFIO.A9 | O.3OO | O.979 |
| IFIO.AII | O.285 | I.OI5 |
| IFIO.BI | O.3O2 | O.86I |
| IFIO.B2 | O.27I | O.952 |
| IFIO.E2 | O.292 | I.OO5 |
| 4AIO.B4 | O.322 | I.279 |
| 3F5.A8 | O.494 | O.773 |
| 3F5.BII | O.563 | O.865 |
| 3F5.D5 | O.658 | O.884 |
| Negative[b] | O.332 | O.295 |

[a]Values are the averages of the absorbance at 405 nm of triplicate samples for each hybridoma subclone in an ELISA.

[b]The value shown is the average absorbance at 405 nm for six wells in an ELISA plate that received culture fluid containing a monoclonal hybridoma antibody specific for dinitrophenol instead of culture fluid containing a mercuric ion-specific monoclonal antibody in the first step of the assay.

Based on this ELISA assay work, hybridomas IFIO and 4AIO were further evaluated to determine if the antibodies secreted were specific for mercuric ions.

2. Determination of Mercuric-ion Specific Monoclonal Antibodies

Various methods were used to confirm that the antibodies secreted by hybridomas 4AIO and IFIO were specific to mercuric ions. If the antibody being secreted by these hybridomas were specific, it should be possible to inhibit binding of the antibody to glutathione-mercuric ions by incubation in the presence of various concentrations of mercuric chloride. This competitive inhibition assay was conducted with antibody-containing culture fluids from the parental hybridomas 4AIO and IFIO. The results for inhibition of IFIO by mercuric chloride and magnesium chloride are shown in Figure I.

Figure I shows inhibition of binding of antibody secreted by hybridoma designated as IFIO to immobilized glutathione-mercuric ion by various concentrations of magnesium ions. Metal ions at the indicated concentrations were incubated with culture fluid from the monoclonal antibody in an enzyme-linked immunosorbent assay ("ELISA") plate. The absorbance at 4O5 nm was determined for each sample, and the percent inhibition of each metal ion concentration was determined by the following formula (X100%):

Percent inhibition is determined by:

$$\frac{A_{405} \text{ of inhibitor} - A_{405} \text{ of neg. control}}{A_{405} \text{ of pos. control} - A_{405} \text{ of neg. control}}$$

Magnesium chloride at the same concentrations as mercuric chloride was included as a control to rule out the possibility that inhibition could be due to excess amounts of divalent cations or increased ionic strength of the incubation solution. It can be seen that 5O% inhibition with mercuric chloride occurs between O.OOOI and O.OOOOI M, while magnesium chloride approaches 5O% inhibition only at the highest concentration.

Therefore, in both enzyme-linked immunosorbent assay (ELISA) and the competitive assay, the monoclonal antibodies were specific for mercuric ions. The preformation of a mercuric ion coordinate covalent complex is not a requirement for monoclonal antibody recognition of mercuric ion. Thus, the monoclonal antibody reacts with free mercuric ions which are independent of coordinating agents.

Various other metals were assayed for inhibition of binding of the monoclonal antibodies to mercuric ion. The cationic metals assayed include the ions of zinc, copper, cadmium, nickel, and arsenic. The results of these inhibition assays are shown in Figure 2. To produce these results the binding of monoclonal antibody secreted by the hybridoma designated as IFIO to immobilized glutathione-mercuric ions by various concentrations of divalent cations was examined. Metal ions at the indicated concentrations were incubated with culture fluid from the antibody in an ELISA plate. The absorbance at 4O5 nm was determined for each sample, and the percent inhibition of each metal ion concentration was determined by the same formula used for Figure I.

However, none of the metals showed a titratable inhibition of monoclonal antibody binding similar to that seen with free mercuric ions. Therefore, based upon the heavy metal ions tested, the monoclonal antibodies produced by immunization with mercuric ions are specific for mercuric ions.

Further analysis shows that the monoclonal antibodies produced are specific for the mercuric ions per se and that glutathione is not needed for the monoclonal antibodies to react with and bind to the mercuric ions. The monoclonal antibody from hybridoma IFIO was assayed against BSA-glutathione, BSA-glutathione mercuric ions, and BSA-mercuric ions. When compared against a negative control consisting of a monoclonal antibody specific for an unrelated antigen the results show that the monoclonal antibody binds to mercuric ion in the absence of glutathione.

BSA-glutathione adsorbed to the wells of a microliter plate effectively binds mercuric ions from solution and enables detection of mercuric ions in a concentration as low as $IO^{-9}$ M (0.2 ppb) by the antibody (Table 3) without appreciable loss of sensitivity.

TABLE 3

| Assay Utilizing BSA-Glutathione Added to Polyvinyl Chloride Microtiter Plates | |
| --- | --- |
| Hg Conc. (M)[a] | A4O5 |
| $10^{-1b}$ | 0.442 |
| $10^{-2}$ | 1.213 |
| $10^{-3}$ | 1.453 |
| $10^{-4}$ | 0.936 |
| $10^{-5}$ | 1.364 |
| $10^{-6}$ | 0.962 |
| $10^{-7}$ | 1.113 |
| $10^{-8}$ | 1.113 |
| $10^{-9}$ | 1.107 |
| 0 | 0.394 |

[a]Mercuric ion concentration refers to the concentration of mercuric chloride in the PBS added to the well to which BSA-glutathione had been absorbed.
[b]The absorbance at concentrations of $10^{-1}$ M is only slightly higher than the control because the large numbers of ions present creates a substantial amount of stearic hindrance which prevents binding and is not evidence of any lack of specificity of the monoclonal antibody.

The specificity of the antibody reactivity for mercuric ion is shown in Figure 3. Here the reactions of various coordinated heavy metal ions with the monoclonal antibody secreted by the hybridoma designated IO indicate that it is specific for mercuric ions.

Phosphate-buffered saline ("PBS") containing metal ions at the indicated concentrations was added to triplicate microtiter wells to which BSA-glutathione had been absorbed. After incubation at room temperature for 3O minutes, the plates were washed to remove unbound metals, and the plates were used for the standard ELISA to detect mercuric ions. In this experiment various heavy metal ions at the indicated concentrations were added to microtiter plates to which BSA-glutathione had been adsorbed. The PBS containing the metal ions was allowed to incubate at room temperature for 3O minutes, and the plates were then used in an ELISA to determine whether the monoclonal antibody would react with the bound metal. The data in Figure 3 show that mercuric ion is the only heavy metal ion which demonstrates a reasonable increase in absorbance.

## B. Particular Preparations

### 1. Linkage of Mercuric Ions to Protein Carriers

To prepare antigen for injection and immunoassay, 136 mg HgCl$_2$ (400 umoles), 61 mg glutathione (200 umoles) and 54 mg NaCl were dissolved in 10 ml of water. Thirty milliliters of cold ethanol were added and incubated for 30 minutes at 0°C. The reaction mixture was centrifuged at 10,000 g for 30 minutes, and the pellet was washed with 30 ml of cold ethanol. The pellet was dissolved in 200 ml of 40% dimethylformamide pH 4.8, containing 200 mg of l-ethyl-3-(3-dimethylaminopropyl)-carbodiimide, and 1 g of either bovine serum albumin or keyhole limpet hemocyanin were added to the solution. The reaction mixture was stirred at room temperature overnight. The mixture was then centrifuged as above, resuspended in PBS, and dialyzed overnight at 4°C against 4 liters of PBS.

### 2. Immunization of BALB/c Mice

BALB/c mice received multiple injections of the antigen prepared with 10 ug of protein per injection. The antigen was mercuric ion-glutathione-KLH emulsified in Freund's adjuvant. Complete adjuvant was used for the first two injections, while incomplete adjuvant was used for all subsequent injections. After the fourth injection, a drop of blood from the tail of each mouse was collected separately in 0.5 ml of PBS, and each sample was assayed by ELISA for the presence of antigen-specific antibody. The mice used for hybridoma production received an intraperitoneal injection consisting of 10 ug of antigen in PBS 3-4 days before cell fusion.

### 3. Hybridoma Production

The spleen was removed aseptically from a mouse, and the cells were isolated by placing the spleen in 5 ml of sterile PBS and teasing it with two sterile, 18-gauge hypodermic needles. The cell suspension was added to an empty sterile, conical, 15-ml centrifuge tube and tissue fragments were allowed to settle for 1-2 minutes. The cells still in suspension were placed in a tube similar to that above and centrifuged at 300 g for 10 minutes at room temperature. The cells were then washed 3 times by centrifugation in serum-free DMEM (Dulbecco's modified Eagle's medium). Spleen cells were co-pelleted with P3X63-Ag8.653 myeloma cells at a ratio of 4 spleen cells to 1 myeloma cell. The supernatant fluid was removed, and the pellet was suspended in 1 ml of 35% polyethylene glycol for 1 minute. The polyethylene glycol was gradually diluted by addition of increasing amounts of serum-free DMEM over a period of 15 minutes. The cells were then suspended in HAT medium (Monoclonal Antibodies, Kennett, McKean, Backitt, eds. Plenum press 1981) at a concentration of 2 X 10$^5$ myeloma cells per ml, and 4 drops from a 5-ml pipet were added to each well of 5 96-well microtiter plates. The plates were incubated in 10% CO$_2$ at 37°C for one week. At that time half of the culture fluid was withdrawn from each well and replaced by 2 drops of fresh HT medium (HAT medium without aminopterin), and the plates were incubated as above for another week. Then, approximately 100 ul of culture fluid was taken from each well containing macroscopically visible cell growth, and the ELISA technique described infra was used for identification of those culture fluids containing mercuric ion-specific antibodies.

### 4. Enzyme-Linked Immunosorbent Assay (ELISA)

Polyvinyl chloride microtiter assay plates were coated with antigen by addition of 50 ul of mercuric ion-glutathione-BSA or glutathione-BSA at a concentration of 5 ug/ml in PBS to each well of the plate. The plates were allowed to incubate at room temperature overnight to allow the antigen to dry on the plate. Next day the plates were blocked by addition of 200 ul of 5% nonfat dry milk in PBS to each well; the addition of the dry milk blocked the remaining protein-binding sites. The plates were incubated for 2 hours at room temperature, then washed 3 times with ELISA wash (PBS with 0.1% of Nonidet P-40).

Fifty microliters of culture fluid being assayed for the presence of antigen-specific antibody were added to the appropriate well, and the plates were incubated at room temperature for 2 hours. The plates were again washed 3 times with ELISA wash, and 50 ul of goat anti-mouse serum (Cooper Biomedical) diluted 1:1000 in 2% BSA in PBS were added to each well. After incubation and washing as above, 50 ul of rabbit anti-goat serum conjugated to alkaline phosphatase (Sigma) diluted 1:1000 in 50 mM Tris-HCl, pH 8.0, containing 1 mM MgCl$_2$, 5% BSA and 0.04% NaN$_3$, were added to each well. After being incubated and washed as above, 150 ul of phosphatase substrate (0.4 mM dinitrophenol phosphate in 1 M diethanolamine, pH 9.8, containing 25 mM MgCl$_2$) were added to each well.

The enzyme catalyzed conversion of dinitrophenol phosphate to dinitrophenol was allowed to proceed at room temperature for 3O-6O minutes. The absorbance of each well at 4O5 nm (dinitrophenol) was measured with a UV spectrometer.

The use of other enzymes as sensors is also possible provided that such enzymes can be linked to an appropriate antibody, and catalyze a reaction which produces a color change. For example, beta galactosidase, urease, carbonic anhydrase or horseradish peroxidase could be utilized in this context.

### 5. Inhibition of Binding of Mercuric ion-Specific Antibody by Metals

Microtiter assay plates containing mercuric ion-glutathione-BSA were prepared as described above. After blocking the plates with non-fat dry milk, 25 ul of a solution containing a known concentration of the metal to be assayed were added to each of triplicate wells of the plate, along with 25 ul of culture fluid containing mercury-specific antibody. The concentrations of metal ranged from 2 X 10$^{-1}$ M to 2 X 10$^{-6}$ M, so the final concentration of metal in the wells ranged from 10$^{-1}$ M to 10$^{-6}$ M. The plates were incubated for 3O minutes at room temperature, washed with ELISA wash as above, and then assayed using the ELISA technique as described above. The absorbance at 4O5 nm was measured for each well, and the percent inhibition of antibody binding for each concentration of metal was calculated according to the following formula (X100%):

Percent inhibition is determined by:

$$\frac{A_{405} \text{ of inhibitor} - A_{405} \text{ of neg. control}}{A_{405} \text{ of pos. control} - A_{405} \text{ of neg. control}}$$

The negative control measured the binding of a dinitrophenol specific antibody to the antigen mentioned above in the presence of the corresponding metal ions. The positive control consisted of triplicate wells that contained 25 ul of mercuric ion-specific antibody and 25 ul of PBS with no metal.

### 6. Binding of Mercuric ions to Immobilized Coordinating Spacer Arms

One hundred microliters of BSA-glutathione at a concentration of 5 ug/ml were added to the wells of a microtiter plate and allowed to dry overnight. The plates were then blocked with nonfat dry milk as above. One hundred microliters of PBS containing a known concentration of the metal ion to be assayed were added to triplicate wells on the plate, which was then incubated at room temperature for 3O minutes. After this incubation period the plates were washed with ELISA wash to remove unbound metal ions and then used in the standard ELISA to measure reactivity with the mercuric ion-specific antibody.

### 7. Assay of Mercuric Ion-Specific Antibody Against BSA Glutathione, BSA Glutathione-Mercury and BSA-Mercury

Mercuric ion specific antibody secreted from hybridoma IFIO was assayed against BSA-glutathione, BSA-glutathione-mercury and BSA-mercuric ions. The results set forth below are the average absorbance plus the standard deviation of nine individual samples assayed against the three antigens.

| Antigen | 1FIO.All | Neg. Control |
|---|---|---|
| BSA-glutathione | 0.4l8 ± O.Ol4 | 0.4l9 ± O.O6l |
| BSA-glutathione-mercuric ion | 3.144 ± 0.132 | 0.171 ± 0.042 |
| BSA-mercuric ion | 2.86l ± O.O92 | 0.223 ± 0.027 |

## Example 2

### Lead Cation Monoclonal Antibodies

Pursuant to the procedures (part A) to generate hybridomas, and to make particular preparations (part B) given in Example 1, monoclonal antibodies to lead cation were produced. The following substitutions in those procedures and preparations were made:

A1) Generation of Hybridomas; lead cations instead of mercuric ion were used; 100 hybridomas were isolated; one showed specific lead cation reactivity.

A2) Determination of Specific Monoclonal Antibodies not conducted.

B1) Linkage; 400 umoles of lead chloride was substituted for the mercuric chloride.

B2) Immunization; The antigen was lead cation-glutathione-KLH.

B3) Hybridoma Production; The same procedure was used.

### B4) Assay

Polyvinyl chloride microtiter assay plates were coated with antigen by addition of 5O ul of lead cation glutathione-BSA or glutathione-BSA at a concentration of 5 ug/ml in PBS to each well of the plate. The plates were allowed to incubate at room temperature overnight to allow the antigen to dry on the plate. Next day the plates were blocked by addition of 2OO ul of 5% nonfat dry milk in PBS to each well; the addition of the dry milk blocked the remaining protein-binding sites. The plates were incubated for 2 hours at room temperature, then washed 3 times with ELISA wash (PBS with O.l% of Nonidet P-4O).

Fifty microliters of culture fluid being assayed for the presence of antigen-specific antibody were added to the appropriate well, and the plates were incubated at room temperature for 2 hours. The plates were again washed 3 times with ELISA wash, and 5O ul of goat anti-mouse serum (Cooper Biomedical) diluted l:lOOO in 2% BSA in PBS were added to each well. After incubation and washing as above, 5O ul of rabbit anti-goat serum conjugated to alkaline phosphatase (Sigma) diluted 1:lOOO in 5O mM Tris-HCl, pH 8.O, containing l mM $MgCl_2$, 5% BSA and O.O4% $NaN_3$, were added to each well. After being incubated and washed as above, l5O ul of phosphatase substrate (O.4 mM dinitrophenol phosphate in l M diethanolamine, pH 9.8, containing 25 mM $MgCl_2$) were added to each well.

The enzyme catalyzed conversion of dinitrophenol phosphate to dinitrophenol was allowed to proceed at room temperature for 3O-6O minutes. The absorbance of each well at 4O5 nm (dinitrophenol) was measured with a UV spectrometer.

The use of other enzymes as sensors is also possible provided that such enzymes can be linked to an appropriate antibody, and catalyze a reaction which produces a color change. For example, beta galactosidase urease, or horseradish peroxidase could be utilized in this context.

These measurements indicated that hybridoma 3H7, produced monoclonal antibodies that were strongly positive against glutathione-lead cation but were negative against glutathione alone.

## Example 3

### Experimental Procedure for Organic Small Moiety

#### 1. Linkage of Trichloroethylene Mimic to Protein Carrier

To prepare antigen for injection and immunoassay, 136 (400 umoles) of N-(trichloroacryloyl)glycine and 54 mg NaCl can be dissolved in 200 ml of 40% dimethylformanide pH 4.8, containing 200 mg of 1-ethyl-3-(3-dimethylaminopropyl)-carbodiimide, and 1 g of either bovine serum albumin or keyhole limpet hemocyanin. The reaction mixture can be stirred at room temperature overnight. The mixture may then be centrifuged resuspended in PBS, and can be dialyzed overnight at 4°C against 4 liters of PBS.

#### 2. Immunization of BALB/c Mice

BALB/c mice can receive multiple injections of the foregoing antigen prepared with 10 ug of protein per injection. After the fourth injection, a drop of blood from the tail of each mouse can be collected separately in 0.5 ml of PBS, and each sample can be assayed by ELISA for the presence of antigen-specific antibody. The mice to be used for hybridoma production can receive an intraperitoneal injection consisting of 10 ug of antigen in PBS 3-4 days before cell fusion.

### 3. Hybridoma Production

The spleen can be removed aseptically from a mouse, and the cells isolated as described above. Spleen cells can be co-pelleted with P3X63-Ag8.653 myeloma cells at a ratio of 4 spleen cells to 1 myeloma cell. The supernatant fluid can be removed, and the pellet suspended in 1 ml of 35% polyethylene glycol for 1 minute. The polyethylene glycol can be gradually diluted by addition of increasing amounts of serum-free DMEM over a period of 15 minutes. The cells can be then suspended in HAT medium (Monoclonal Antibodies, Kennett, McKean, Backitt, eds. Plenum Press 1981) at a concentration of 2 X $10^5$ myeloma cells per ml, and 4 drops from a 5-ml pipet can be added to each well of 5 96-well microtiter plates. The plates can be incubated as described above. Approximately 100 ul of culture fluid can be taken from each well containing macroscopically visible cell growth, and the ELISA technique described above can be used for identification of those desirable culture fluids.

### 4. Enzyme-Linked Immunosorbent Assay (ELISA)

Polyvinyl chloride microtiter assay plates can be coated with antigen by addition of 50 ul of N-(trichloroacryloyl)- glycine-BSA or glycine BSA at a concentration of 5 ug/ml in PBS to each well of the plate. The plates can be incubated and prepared for ELISA assay as described above.

Fifty microliters of culture fluid being assayed for the presence of antigen-specific antibody can added to the appropriate well, and the plates can be incubated at room temperature for 2 hours. The plates can again be washed with ELISA wash, and 50 ul of goat anti-mouse serum (Cooper Biomedical) diluted 1:1000 in 2% BSA in PBS can be added to each well. After incubation and washing as above, 50 ul of rabbit anti-goat serum conjugated to alkaline phosphatase (Sigma) diluted 1:1000 in 50 mM Tris-HCl, pH 8.0, containing 1 mM $MgCl_2$, and 0.04% $NaN_3$, can be added to each well. After being incubated and washed as above, 150 ul of phosphatase substrate (0.4 mM dinitrophenol phosphate in 1 M diethanolamine, pH 9.8, containing 25 mM $Mg_2$ Cl) can be added to each well.

The enzyme-catalyzed conversion of dinitrophenol phosphate to dinitrophenol may be allowed to proceed at room temperature for a sufficient time. Then, the absorbance of each well at 405 nm (dinitrophenol) can be measured with a spectrophotometer. Appropriate absorbance from the wells containing immunogen compound and lack of absorbance from corresponding wells containing carrier will select the desired culture fluid and hybridoma.

### Assay for Trichloroethylene in Water

Microtiter assay plates coated with a constant amount of immobilized monoclonal antibody from the foregoing selected hybridoma can be prepared by the technique of L. Wide and J. Porath, Biochem. Biophys. Act A, 130: 257-260 (1966). Briefly, this technique will involve addition of 50$\mu$l of metal-specific antibody (20 mg/ml in PBS) to each well of a microtiter plate. The plate will be incubated for 2hrs., washed with PBS, and blocked for 2 hrs. with 5% nonfat dry milk. The plates can then be saturated with radioactive trichloroethylene ($^{14}$C or $^3$H). The plates can be incubated for a sufficient time, about 1 hour and then washed with the ELISA wash described above. Addition of aliquots of water containing an unknown concentration of trichloroethylene can then be added to the assay plate wells. After incubating for a sufficient time, e.g. about 1, hour, the fluids from each of the wells can be removed and counted in a scintillation counter. A comparison of the counts with a standard curve constructed from data produced by tests on known concentrations of trichloroethylene will yield the concentration of unknown in the test sample.

### Example 4

**Procedure for N-blocking Groups for Amino Acids**

Over 80% of the soluble proteins in biological cells including those of plants possess amino termini that are not free but chemically blocked. Determining the identity of the blocked amino groups of proteins is extremely important in understanding the biological activities of the physiologically important protein molecules. However, only a handful of these blocked amino groups have been structurally determined because the currently available HPLC-mass spectrometric method is difficult, tedious and extremely expensive. The present invention permits production of a set of monoclonal antibodies specifically designed to determine the terminal amines of proteins, both efficiently and economically. Once such monoclonal antibodies are produced, these antibodies can be made commercially available as specialty reagent chemicals for the determination of protein structure. Pursuant to the techniques described above two monoclonal antibodies have been produced that are specifically designed to detect N-acetyl group and/or N-acetyl amino acid, the acetyl group being most common amino-blocking group in proteins.

Identification of the amino terminal group in phytochrome is an example of the difficulties mentioned above. It is blocked due to the posttranslational modification. However, conventional Edman degradation does not reveal the chemical identity of the blocked amino terminal group in the protein and the amino acid sequencing deduced from cDNA codes is not useful, since the nature of posttranslational modifications are not determinable from the DNA base sequence. Identification of the amino terminal groups in protein is tedious and requires a substantial amount of chromatographically purified N-terminal peptides, usually by ion exchange column chromatography or HPLC, for mass spectrometric analysis. Even in a mass spectrometric analysis, 50-100 nmol of purified peptide is required. In this respect, characterization of the blocked amino terminal groups in proteins is facilitated by the recent development of tandem mass spectrometry for relatively large peptides of 10 or more amino acid residues. In spite of this and other developments, most of the blocked N-terminus groups in a number of proteins remains to be analyzed. The present invention contemplates a set of monoclonal antibodies specifically targeted for various N-blocking groups for terminal amino acids such as those listed in the following table:

| Blocking Group | Occurrence(example) | Reference |
|---|---|---|
| N-acetyl | ovalbumin | Tsunasawa & Narita(1983) |
| Pyrrolidone carboxyl | immunoglobulins | Wold(1981) |
| Fatty acyl | E. coli murein lipoproteins | Wold(1981) |
| N-acetyl and O-phosphoryl (N-ser) | human lipocortin(I) | Bieman & Scoble(1987) |
| N-acetylglucosaminyl | N-terminal serine | Wold(1981) |
| O-phosphoryl | N-terminal serine | Wold(1981) |
| O-mannosyl | N-terminal serine | Wold(1981) |
| Pyruvate | N-terminal serine | Wold(1981) |
| α-ketobutyrate | N-terminal threonine | Wold(1981) |
| O-mannosyl | N-terminal threonine | Wold(1981) |
| O-adenosyl | N-terminal tyrosine | Wold(1981) |

The first step in production of monoclonal antibodies to an N-block group on an amino acid is the attachment of this molecule (the hapten) to a larger carrier molecule. The haptens are N-acetyl blocked amino acids and the carrier molecule is keyhole limpet hemocyanin (KLH). This attachment has been accomplished to a degree of about 15 haptens per 100,000 kDa of protein.

The attachment was performed via a dicyclohexylcarbodiimide mediated synthesis of the N-hydroxysuccinimide activated ester of the amino acid. An undesirable side reaction involving polyester formation through reaction of the hydroxyl of the serine activated ester is avoided by using the N-acetyl serine activated ester immediately after synthesis and not concentrating the synthesis product.

The activated ester was then attached to KLH at pH 7.0 in aqueous solution. The attachment resulted with about 60% blockage of exposed lysine residues as determined by a TNBS assay. Pursuant to the immunization procedure discussed above, the N-acetyl serine conjugated KLH was injected and booster shots given. The resulting serum showed preferential binding to N-acetyl serine conjugated BSA vs. unconjugated BSA. Pursuant to the hybridoma formation procedure discussed above, cell fusions were performed to obtain N-acetyl serine specific antibodies. Two monoclonals were isolated which reacted specifically to the hapten conjugated BSA. The first antibody bound N-acetyl serine conjugated BSA more than 20 times stronger (by ELISA) than N-acetyl glycine conjugated BSA. (The N-acetyl glycine BSA synthesis was performed in the same manner as the N-acetyl serine BSA synthesis). The second monoclonal showed only a slightly higher binding preference for N-acetyl serine BSA over N-acetyl glycine BSA (1.4 times greater reaction, ELISA determination).

**Claims**

1. A monoclonal antibody displaying immunoreactivity with a small moiety, which is produced by a hybridoma of myeloma immortal cells and mammalian immune cells sensitized against an immunogen compound comprising a protein carrier, a spacer arm covalently bonded to the carrier the small moiety coordinated in a partially exposed state with the spacer arm, and the spacer arm being semi-rigid and having a small moiety coordination site arranged to maintain the small moiety in at least the partially exposed state, wherein the small moiety is an organic compound having a molecular size of no more than about 15 atoms in length and is a linear or branched aliphatic molecule or a small peptide.

2. A monoclonal antibody according to claim 1 wherein the dissociation constant for the particular organic compound is at least about $10^{-6}$.

3. A fragment of a monoclonal antibody of any of claims 1 or 2.

4. A hybridoma of myeloma immortal cells and mammalian immune cells from a mammel previously immunized with an immunogen compound, wherein:
the immunogen compound is a protein carrier, a spacer arm covalently bonded to the carrier and a small moiety coordinated in at least a partially exposed state with the spacer arm; and the hybridoma produces a monoclonal antibody which immunoreacts with the small moiety, wherein the small moiety is an organic compound having a molecular size of no more than about 15 atoms in length and is a linear or branched aliphatic molecule or a small peptide.

5. A hybridoma according to claim 4 which is selected by the reactivity of its culture fluid toward the small moiety or immunogen compound and its lack of reactivity toward the corresponding carrier-spacer arm compound.

6. An immunogen compound comprising a protein carrier, a spacer arm covalently bonded to the carrier and a small moiety coordinated in at least a partially exposed state with the spacer arm, the small moiety being an organic molecule having a molecular size of no more than about 15 atoms in length.

7. An immunogen compound according to claim 6 wherein the organic molecule is a linear or branched aliphatic molecule or a small peptide.

8. An immunogen according to claim 6 wherein the spacer arm is semi-rigid and has a small moiety coordination site arranged to maintain the small moiety in at least a partially exposed state.

9. An immunogen compound according to claim 6 wherein the spacer arm is an oligopeptide or aliphatic compound substituted with no more than about two pendent Lewis acid or base groups and a coupling group for forming a covalent bond with the protein carrier, or is an aliphatic fragment having a coupling group for forming a covalent bond with the protein carrier and being adapted for carbon-carbon bonding with, or covalently carbon bonded to the moiety, said coupling group being an amino, carboxylic acid, hydroxyl, mercapto or aldehyde group, and said spacer arm being no more than about 25 atoms in length.

10. A method for detecting a small moiety in liquid media, which comprises:
combining an aliquot of a liquid mixture suspected of containing the small moiety and an excess of immobilized or immobilizable antigen probe to produce a coordinated immobilized small moiety mixture;
combining the coordinated immobilized small moiety mixture and an excess of a monoclonal antibody of claim 1 to form a monoclonal antibody-antigen complex; and
determining the amount of monoclonal antibody-antigen complex present, wherein the small moiety is an organic compound having a molecular size of no more than about 15 atoms in length and is a linear or branched aliphatic molecule or a small peptide.

11. A method for detecting a small moiety in liquid, which comprises: saturating a known first amount of an immobilized monoclonal antibody of claim 1 with a known amount of tagged small moiety to form a mixture containing a tagged monoclonal antibody-antigen complex;
removing all uncomplexed components from the mixture;
adding an aliquot of an unknown second amount of small moiety to the mixture and allowing the resulting reaction to equilibrate; and
determining the concentration of unknown small moiety by measuring the amount of tag in solution after equilibration, wherein the small moiety is an organic compound having a molecular size of no more than about 15 atoms in length and is a linear or branched aliphatic molecule or a small peptide.

**12.** A method for removing a small moiety from a liquid, which comprises:

adding an effective amount of monoclonal antibody of claim 1 to the liquid to form an immunoconjugate; and

removing the immunoconjugate from the liquid, wherein the small moiety is an organic compound having a molecular size of no more than about 15 atoms in length and is a linear or branched aliphatic molecule or a small peptide.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A1-2 561 660 (CENTRE NATIONAL DE LA RECHERCHE SCIENTIFIQUE (CNRS) ET AL) | 6-9 | C 12 P 21/08<br>C 12 N 5/16<br>G 01 N 33/53<br>G 01 N 33/84<br>C 07 K 15/28 |
| Y | * see claims 7-15 *<br>--- | 1-12 | |
| X | NATIONAL LIBRARY OF MEDICINE, File Medline, NLM Accession no. 88013838, BUENAFE A.C.: "Combining site specificity of monoclonal antibodies to the organo-phosphate hapten soman", & Mol Immunol 1987 May;24(5):401-7<br>--- | 1 | |
| X | DIALOG INFORMATION SERVICES, File 351, World Patent Index, Dialog accession no. 007104778,((NICI) NICHIEI KAGAKU KOG KK), "New monoclonal anti-phosphoryl choline antibody used to diagnose malignant tumours partic lung carcinoma", & JP 62051699, A, 870306, 8715<br>--- | 1,4,6 | |
| X | DIALOG INFORMATION SERVICES, File 154, Medline, Dialog accession no. 05398874, CHEDID L et al: "Specific absorption with monoclonal antibodies to muramyl dipeptide of the pyrogenic and somno-genic activities of rabbit monokine",& Proc Natl Acad Sci U S A Sep 1984, 81(18) p5888-91<br>--- | 1,4,6 | TECHNICAL FIELDS SEARCHED (Int. Cl.4)<br><br>G 01 N<br>C 12 P<br>C 07 K |
| X | WO-A1-8 602 736 (EKS, INC.)<br>* see claims 16-17<br>--- | 1,4,6,7 | |

-/-

The present search report has been drawn up for all claims

| Place of search | Date of completion of the search | Examiner |
|---|---|---|
| STOCKHOLM | 10 DECEMBER 1993 | GUSTAFSSON C-O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| X | CHEM. PHARM. BULL., vol. 33, no. 7, 1985, pages 2871-2876, MIKIO OHNISHI et al: "Water-Soluble Carrier Proteins Having Carboxyl Spacer Groups", | 6-9 | C 12 P 21/08<br>C 12 N 5/16<br>G 01 N 33/53<br>G 01 N 33/84<br>C 07 K 15/28 |
| Y | * see "Results and Discussion *<br>--- | 1-12 | |
| X | EP-A1-043 285 (DAINIPPON PHARMACEUTICAL CO., LTD.) | 6-9 | |
| Y | * see table 2 *<br>--- | 1-12 | |
| X | EP-A2-028 795 (MILES LABORATORIES INC.)<br>* see pages 7-10 *<br>--- | 6-9 | |
| X | PATENT ABSTRACTS OF JAPAN, vol. 7, no. 121 abstract of JP 58-38860 (A), 7 March 1983, (DAINIPPON SEIYAKU K.K. et al)<br>--- | 6-9 | TECHNICAL FIELDS SEARCHED (Int. Cl.4)<br><br>G 01 N<br>C 12 P<br>C 07 K |
| X | PATENT ABSTRACTS OF JAPAN, vol. 4, no. 149 abstract of JP 55-99072 (A), 28 July 1980, (MITSUBISHI YUKA K.K. et al)<br>--- | 6-9 | |
| X | EP-A2-261 416 (BATTELLE-INSTITUT E.V.)<br>--- | 6-11 | |
| X | DE-A1-35 15 901 (BATTELLE-INSTITUT E.V.)<br>--- | 6-9 | |
| X | EP-A2-149 405 (CENTRE NATIONAL DE LA RECHERCHE SCIENTIFIQUE (CNRS) ET AL) | 6-9 | |
| Y | * page 4, lines 35-39, claims 7-10 *<br>--- | 1-12 | |
| Y | METHODS IN ENZYMOLOGY, vol. 70, 1980, pages 85-104, BERNARD F. ERLANGER: "The Preparation of Antigenic Hapten- | 1-12 | |
| X | -Carrier Conjugates: A Surveye",<br>--- | 6-9 | |
| A | MONOCLONAL ANTIBODIES AND NEW TRENDS IN IMMUNOASSAYS, Ch. A. Bizollon editor, 1984, pages 52-59, M DELAAGE et al: "Monoclonal antibodies to haptens",<br>---<br>----- | 1-12 | |